# EUROPEAN PATENT APPLICATION

(11) **EP 3 991 643 A1**
(43) Date of publication of application: **04.05.2022**
(21) Application number: 20833267.6
(22) Date of filing: 24.06.2020
(51) Int. Cl.: A61B 5/02, A61B 5/1455

(54) **BLOOD COMPONENT MEASUREMENT SYSTEM, BLOOD COMPONENT MEASUREMENT METHOD, AND BLOOD COMPONENT MEASUREMENT PROGRAM**

(30) Priority: 25.06.2019 JP 2019117598
(71) Applicant: KOWA COMPANY, LTD., Naka-ku Nagoya-shi Aichi 460-8625 (JP)
(72) Inventor: YABUSAKI, Katsumi, Higashimurayama-shi, Tokyo 189-0022 (JP); KATO, Nakahiro, Higashimurayama-shi, Tokyo 189-0022 (JP); SUZUKI, Miyako, Higashimurayama-shi, Tokyo 189-0022 (JP)
(74) Representative: Ribeiro, James Michael
(86) International application number: PCT/JP2020/024736
(87) International publication number: WO 2020/262431

(57) **Abstract**

An object of the present invention is to provide a technique that can improve the measurement accuracy of the blood component concentration. A blood component measurement system emits light to a measurement site of a living body while the measurement site is compressed, and calculates a concentration of a predetermined blood component based on a pulse wave signal acquired based on light received from the measurement site. A blood component measurement system includes a processing unit that performs a process of analyzing the pulse wave signal and determining a pressure condition of the measurement site; and a calculation unit that calculates a concentration of the blood component using the pulse wave signal in a period in which the pressure condition is determined.

## Description

### [Field]

The present invention relates to a blood component measurement system, a blood component measurement method, and a blood component measurement program.

### [Background]

There is a technique in which light is emitted to a measurement site of a living body, a pulse wave signal is acquired based on the light received from the measurement site, and the blood component concentration is measured from the pulse wave signal. Patent Literature 1 discloses a technique in which, when a measurement site of a living body is pressed, light is emitted to the measurement site to detect a volume pulse wave, a pressing force at which the amplitude of the volume pulse wave has a maximum value is determined as an optimal pressing force, and measurement light is then emitted to the measurement site pressed with the optimal pressing force, and a blood glucose level is calculated based on the light reception results.

### [Citation List]

### [Patent Literature]

[PTL 1] JP-A-2016-112042

### [Summary]

### [Technical Problem]

Pulse waves are affected by the diurnal rhythm and change from moment to moment. The measurement result of the pulse wave signal changes according to a magnitude of a force with which the measurement site is compressed, and if this force is too strong or too weak, a favorable pulse wave signal cannot be obtained. In addition, when an optimal pressing force for the measurement site is determined, a pulse wave signal is then acquired, and the blood component concentration is measured, for example, if the pressing force changes during measurement due to an external impact or the like, it is not possible to respond to the change in pressing force during measurement, and it is not possible to acquire an accurate pulse wave signal. If an accurate pulse wave signal cannot be acquired, the measurement accuracy of the blood component concentration decreases.

In view of the above circumstances, an object of the present disclosure is to provide a technique that can improve the measurement accuracy of the blood component concentration.

### [Solution to Problem]

A blood component measurement system of the present disclosure is a blood component measurement system that emits light to a measurement site of a living body while the measurement site is compressed, and calculates a concentration of a predetermined blood component based on a pulse wave signal acquired based on light received from the measurement site, the system including: a processing unit that performs a process of analyzing the pulse wave signal and determining a pressure condition of the measurement site; and a calculation unit that calculates a concentration of the blood component using the pulse wave signal in a period in which the pressure condition is determined. According to the blood component measurement system of the present disclosure, since a pulse wave signal having an appropriate pressure value for the measurement site can be used for calculating the concentration of the predetermined blood component, it is possible to improve the measurement accuracy of the blood component concentration.

In the blood component measurement system, the calculation unit may calculate a concentration of the blood component selectively using the pulse wave signal acquired in a period in which a pressure value for the measurement site is within a predetermined range.

In addition, in the blood component measurement system, the calculation unit calculates a concentration of the blood component using the pulse wave signal acquired in a predetermined period and calculates a degree of reliability of the concentration of the blood component based on the result of the process during the predetermined period, and the blood component measurement system may further include a notification unit that notifies of the concentration of the blood component when the degree of reliability is equal to or larger than a predetermined value and notifies of re-measurement of the concentration of the blood component when the degree of reliability is less than the predetermined value.

In addition, in the blood component measurement system, the processing unit may determine whether a pressure value for the measurement site is within a predetermined range by performing the process, and continuously perform the process on the pulse wave signal acquired after it is determined that the pressure value for the measurement site is within the predetermined range, and the calculation unit may calculate a concentration of the blood component using the pulse wave signal acquired after the processing unit determines that the pressure value for the measurement site is within the predetermined range.

In addition, the present disclosure can be seen from an aspect of a blood component measurement method or blood component measurement program. For example, the blood component measurement method of the present disclosure is a blood component measurement method in which light is emitted to a measurement site of a living body while the measurement site is compressed, and a concentration of a predetermined blood component is calculated based on a pulse wave signal acquired based on light received from the measurement site, the method including: performing a process of analyzing the pulse wave signal and determining a pressure condition of the measurement site; and calculating a concentration of the blood component using the pulse wave signal in a period in which the pressure condition is determined.

### [Advantageous Effects of Invention]

According to the technique of the present disclosure, it is possible to improve the measurement accuracy of the blood component concentration.

### [Brief Description of Drawings]

[Fig. 1] Fig. 1 is a diagram depicting an example of a configuration of a blood component measurement system according to one embodiment.
[Fig. 2] Fig. 2 is a diagram schematically depicting a pulse wave signal measurement device in the blood component measurement system according to one embodiment.
[Fig. 3] Fig. 3 is a diagram schematically depicting a part of the pulse wave signal measurement device in the blood component measurement system according to one embodiment.
[Fig. 4] Fig. 4 is a diagram schematically depicting a part of the pulse wave signal measurement device in the blood component measurement system according to one embodiment.
[Fig. 5] Fig. 5 is a circuit diagram of a part of the pulse wave signal measurement device in the blood component measurement system according to one embodiment.
[Fig. 6] Fig. 6 is a graph depicting an example of a pulse wave signal.
[Fig. 7] Fig. 7 is a graph illustrating an appropriate range of pressure values for a measurement site.
[Fig. 8] Fig. 8 is a graph illustrating a method of monitoring a pressure value in the blood component measurement system according to one embodiment.
[Fig. 9] Fig. 9 is a graph illustrating frequency analysis of a pulse wave signal in the blood component measurement system according to one embodiment.
[Fig. 10] Fig. 10 is a graph illustrating frequency analysis of a pulse wave signal in the blood component measurement system according to one embodiment.
[Fig. 11] Fig. 11 is a graph illustrating an inflection point count of a pulse wave signal in the blood component measurement system according to one embodiment.
[Fig. 12] Fig. 12 is a graph illustrating a method of comparison with a reference pattern obtained from the shapes of a plurality of beats in the blood component measurement system according to one embodiment.
[Fig. 13] Fig. 13 is a graph illustrating a method of comparison with a sawtooth wave obtained from the shape of a single beat in the blood component measurement system according to one embodiment.
[Fig. 14] Fig. 14 is a graph illustrating a method of comparison with a sawtooth wave obtained from the shape of a single beat in the blood component measurement system according to one embodiment.
[Fig. 15] Fig. 15 is a graph illustrating a method of comparison with a sawtooth wave obtained from the shape of a single beat in the blood component measurement system according to one embodiment.
[Fig. 16] Fig. 16 is a graph illustrating a method of comparison with a sawtooth wave obtained from the shape of a single beat in the blood component measurement system according to one embodiment.
[Fig. 17] Fig. 17 is a flowchart of a blood component measurement process using the blood component measurement system according to one embodiment.

### [Description of Embodiments]

Embodiments of the present invention will be described below with reference to the drawings. Here, configurations of the following embodiments are examples, and the present invention is not limited to the configurations of these embodiments.

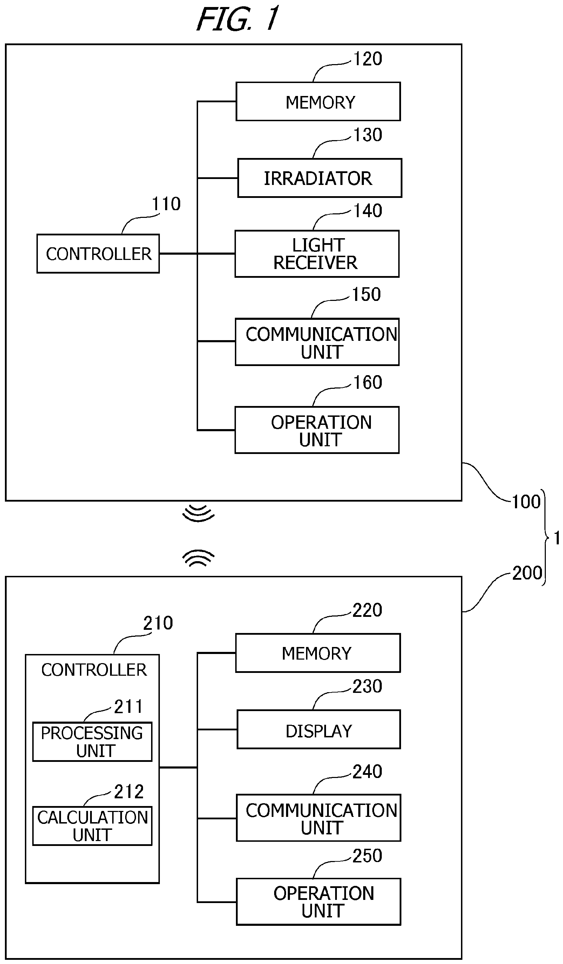

First, a blood component measurement system according to the present embodiment will be described. Fig. 1 is a diagram depicting a schematic configuration of a blood component measurement system 1 according to the present embodiment. As depicted in Fig. 1, the blood component measurement system 1 includes a pulse wave signal measurement device 100 and a terminal device 200.

The pulse wave signal measurement device 100 emits near infrared light from light-emitting diodes (LEDs) to a measurement site, which is a part of a body of a living body containing blood, receives near infrared light that has passed through the blood in the measurement site with a photodiode (PD), and acquires received light data. Since living bodies are not transparent except for the eyeballs, light is not transmitted therethrough. However, for example, light that has entered the inside of a human finger is scattered by tissues, blood, and the like, and does not travel straight, and a small part of the light that has entered reaches the PD and is detected. Among the detected light intensities, the component that fluctuates periodically is a pulse wave signal detected with received light data of the light that has passed through the blood.

Here, a human is exemplified as a living body for which a pulse wave signal is measured by the pulse wave signal measurement device 100. When the measurement target is a human, the measurement sites may be sites in which pulsation can be easily detected with near infrared light, and are preferably fingers, palms, wrists, medial elbows, backs of knees, soles of the feet, toes, ear lobes, fronts of ears, lips, the epigastrium or the like and more preferably the thumb, index finger, or middle finger at which pulsation can be clearly detected. Hereinafter, a human will be described as the measurement target subject and a thumb will be described as the measurement site. Here, the measurement target subject and the measurement site are not limited thereto.

The pulse wave signal measurement device 100 includes a controller 110, a memory 120, an irradiator 130, a light receiver 140, a communication unit 150, and an operation unit 160. The controller 110 includes a central processing unit (CPU) and controls respective units in the pulse wave signal measurement device 100. The memory 120 includes a non-volatile memory such as a flash memory and an electrically erasable programmable read-only memory (EEPROM), and a random access memory (RAM). The memory 120 stores a control program in the pulse wave signal measurement device 100 and data obtained when various processes are executed.

The irradiator 130 emits near infrared light to the measurement site of the living body. In the present embodiment, the pulse wave signal measurement device 100 measures a pulse wave signal when the irradiator 130 emits near infrared light to a human thumb which is the measurement target.

The intensity of light that has passed through the blood in blood vessels of the human finger fluctuates periodically due to blood pulsation. The blood component measurement system 1 according to the present embodiment measures the absorbance of blood at a plurality of wavelengths in a non-invasive manner using a pulse wave signal which is a time-dependent change in the intensity of light that has passed through the blood in blood vessels of the human thumb, and measures a value of triglycerides in blood (hereinafter referred to as a "blood TG value") and a value expressed as a percentage proportion of glycated hemoglobin with respect to the total amount of hemoglobin contained in blood (hereinafter referred to as an "HbAlc value").

When the concentration of the blood TG value increases and the turbidity of blood increases, the absorbance of near infrared light near a wavelength of 1,050 nm increases. Here, in the present embodiment, the blood TG value is measured based on the difference between the absorbance of blood at a wavelength of 1,050 nm and the absorbance of blood at a wavelength of 1,300 nm.

In addition, the inventors of the present invention found that the absorbance near a wavelength of 1,450 nm changes significantly depending on the HbAlc value as compared with other wavelengths. In addition, since the absorbance near a wavelength of 900 nm to 1,300 nm changes depending on the total amount of hemoglobin in blood, the HbAlc value is measured using the absorbance at 1,450 nm and the absorbance at a wavelength of 1,050 nm or a wavelength of 1,300 nm in the present embodiment.

In order to measure the blood TG value and HbAlc value in a non-invasive manner by emitting near infrared light at the above wavelength to a human finger, the irradiator 130 of the pulse wave signal measurement device 100 includes an LED with a peak wavelength of 1,050 nm as a first light-emitting element, an LED with a peak wavelength of 1,300 nm as a second light-emitting element, and an LED with a peak wavelength of 1,450 nm as a third light-emitting element. Here, details of these LEDs and details of a method of measuring the blood TG value and HbAlc value will be described below.

The light receiver 140 receives light that has passed through the blood at the measurement site. The near infrared light emitted by the irradiator 130 passes through the blood contained in the measurement site of the living body and is received by the light receiver 140. The light receiver 140 includes a photodiode (PD), detects light that has passed through the blood with the PD, and outputs an intensity thereof as a voltage signal. In addition, the pulse wave signal measurement device 100 has an analog digital (AD) converter (not depicted), performs AD conversion on an output signal as received light data from the PD of the light receiver 140, and then outputs the result to the controller 110. The controller 110 stores the received light data as a pulse wave signal in the memory 120. Here, the positional relationship between the irradiator 130 and the light receiver 140 will be described below.

The communication unit 150 wirelessly communicates with the terminal device 200 according to known short-range wireless communication such as Bluetooth (registered trademark), Bluetooth Low Energy (BLE), and Wi-Fi. The pulse wave signal measurement device 100 can send various types of data such as a pulse wave signal to the terminal device 200 and receive a control signal from the terminal device 200.

The operation unit 160 is composed of, for example, a button or a touch panel. Turning a power supply on/off and setting communication with the terminal device 200 are performed by operating the operation unit 160.

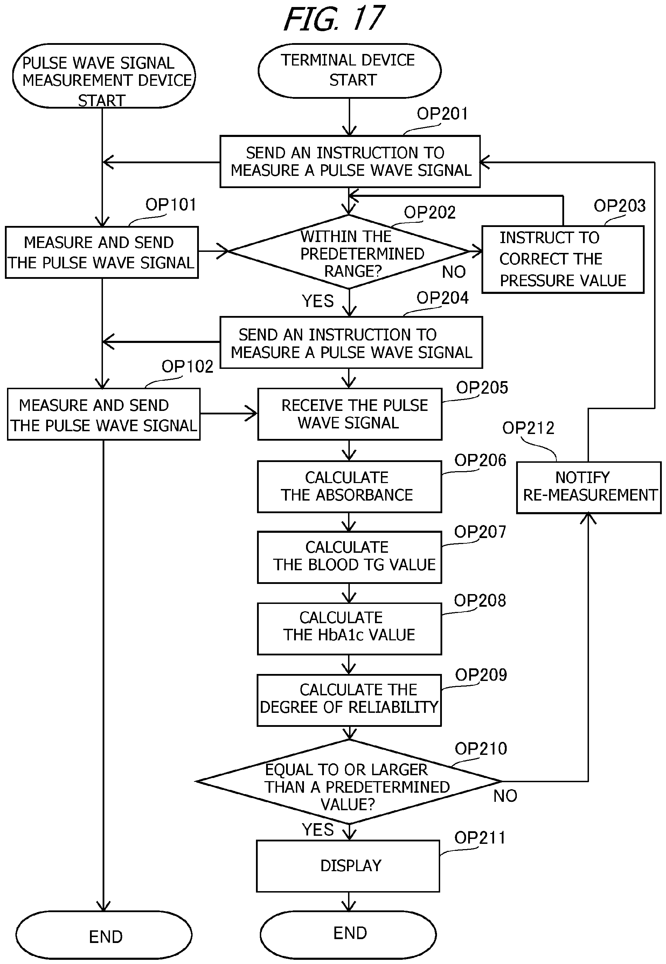

Next, the terminal device 200 of the blood component measurement system 1 according to the present embodiment will be described. Examples of the terminal device 200 include smartphones, feature phones, tablet personal computers, notebook personal computers, desktop personal computers, and other various electronic devices. In addition, the terminal device 200 executes various processes in blood component measurement to be described below (refer to Fig. 17). In the present embodiment, the terminal device 200 is an example of a computer. In the blood component measurement system 1 according to the present embodiment, the terminal device 200 calculates the blood TG value and HbAlc value based on the pulse wave signal acquired by the pulse wave signal measurement device 100.

The terminal device 200 includes a controller 210, a memory 220, a display 230, a communication unit 240, and an operation unit 250. The controller 210 includes a CPU, and controls respective units in the terminal device 200. The memory 220 includes a hard disk drive (HDD), a non-volatile memory such as a flash memory or an EEPROM, and a RAM. The memory 220 stores the blood component measurement program, the control program, and data obtained when various processes are executed in the terminal device 200. In the controller 210, the CPU executes the program stored in the memory 220 and thus respective functional units of a processing unit 211 and a calculation unit 212 are realized. The processing unit 211 performs a process of analyzing the pulse wave signal and determining the pressure condition of the measurement site. The calculation unit 212 calculates the blood component concentration using the pulse wave signal and calculates the degree of reliability of the blood component concentration.

The display 230 (an example of "notification unit") is composed of a liquid crystal display device, an organic EL display device or the like. The terminal device 200 displays the measured blood TG value and HbAlc value and the degree of reliability of the measured value on the display 230.

The communication unit 240 wirelessly communicates with the pulse wave signal measurement device 100 according to known short-range wireless communication such as Bluetooth, BLE, and Wi-Fi, and can receive various types of data such as a pulse wave signal from the pulse wave signal measurement device 100, and can send the control signal to the pulse wave signal measurement device 100.

The operation unit 250 is composed of, for example, a button or a touch panel. Starting of measurement of the pulse wave signal and setting of communication with the pulse wave signal measurement device 100 are performed by operating the operation unit 250.

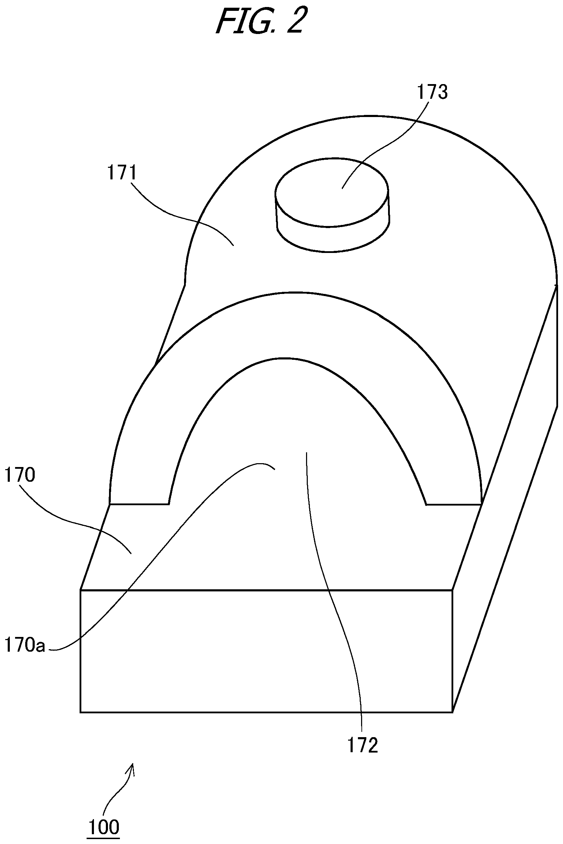

Next, with reference to Fig. 2, a configuration example of the pulse wave signal measurement device 100 according to the present embodiment will be described in more detail. Fig. 2 is an exterior perspective view of the pulse wave signal measurement device 100. The pulse wave signal measurement device 100 includes a housing 170 and an upper part cover 171 that covers the upper part of the housing 170. In addition, in the pulse wave signal measurement device 100, an opening 172 for inserting a subject's finger which is the measurement target is provided between the housing 170 and the upper part cover 171. When the subject's finger is inserted into the opening 172, the irradiator 130 and the light receiver 140 are provided on a contact surface 170a that comes into contact with the finger in the housing 170.

In the upper part cover 171, a knob 173 for adjusting the force with which the subject's finger is pressed against the contact surface 170a is provided. In the present embodiment, the knob 173 is bonded to a screw (not depicted) that penetrates the upper part cover 171. A pressing plate (not depicted) that presses the subject's finger is bonded to the tip of the screw. When the knob 173 is turned to the right, the screw and the pressing plate descend toward the contact surface 170a, and the pressing force with which the pressing plate presses the subject's finger increases. On the other hand, when the knob 173 is turned to the left, the screw and the pressing plate are raised toward the upper part cover 171, and the pressing force with which the pressing plate presses the subject's finger decreases. The subject can adjust the force with which the finger is pressed against the contact surface 170a by turning the knob 173 to the left or right.

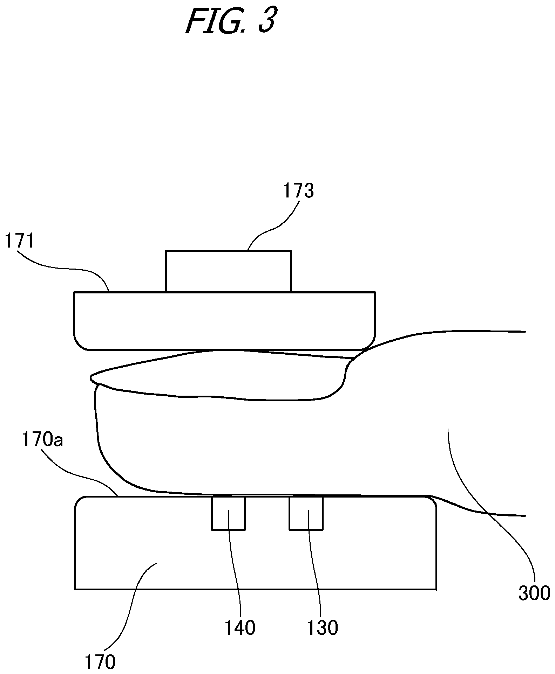

Fig. 3 is a diagram schematically depicting a state in which, in the pulse wave signal measurement device 100 depicted in Fig. 2, the subject inserts a thumb 300 into the opening 172. In the blood component measurement system according to the present embodiment, in the pulse wave signal measurement device 100, a reflected light method in which the irradiator 130 emits light to the ventral side of the thumb 300 and the light that has passed through the blood is received by the light receiver 140 disposed on the ventral side of the finger is used.

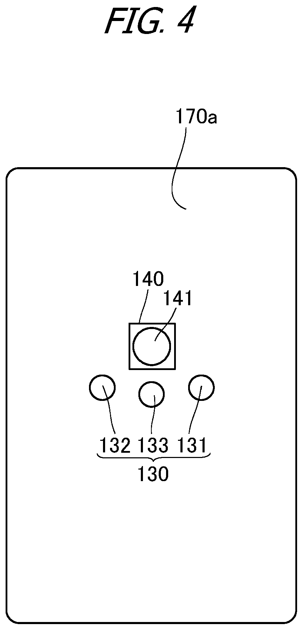

Fig. 4 is a plan view depicting the contact surface 170a of the pulse wave signal measurement device 100 on which the irradiator 130 and the light receiver 140 are disposed. The irradiator 130 includes a first LED 131, a second LED 132, and a third LED 133. The first LED 131 emits light having a peak wavelength at a wavelength of 1,050 nm. The second LED 132 emits light having a peak wavelength at a wavelength of 1,300 nm. The third LED 133 emits light having a peak wavelength at a wavelength of 1,450 nm.

In addition, in the present embodiment, the irradiator 130 emits light with wavelengths in increasing order of biopermeability in the measurement site of the living body. Here, the biopermeability is higher when the absorption rate of light in the living body is lower and is lower when the absorption rate of light in the living body is higher. Generally, near infrared light at a wavelength of 850 nm to 1,500 nm has a low absorption rate in the living body and a high biopermeability. In near infrared light at the above wavelength, the biopermeability in the human finger at a wavelength of 1,450 nm, a wavelength of 1,300 nm, and a wavelength of 1,050 nm, increases in that order. The irradiator 130 emits light in the order of wavelengths with low biopermeability, that is, in the order of the third LED 133, the second LED 132, and the first LED 131, when it is controlled by the controller 110. In addition, as depicted in Fig. 4, the third LED 133 that emits light having a peak wavelength at a wavelength of 1,450 nm, which has relatively low biopermeability among these three wavelengths is disposed at the center of the three LEDs 131 to 133 arranged side by side so that the distance from the light-receiving unit 40 is the shortest.

In addition, the light receiver 140 includes a PD 141. The PD 141 receives light that has been emitted from the irradiator 130 to the finger and has passed through the blood. The PD 141 outputs a voltage signal as received light data upon receiving light.

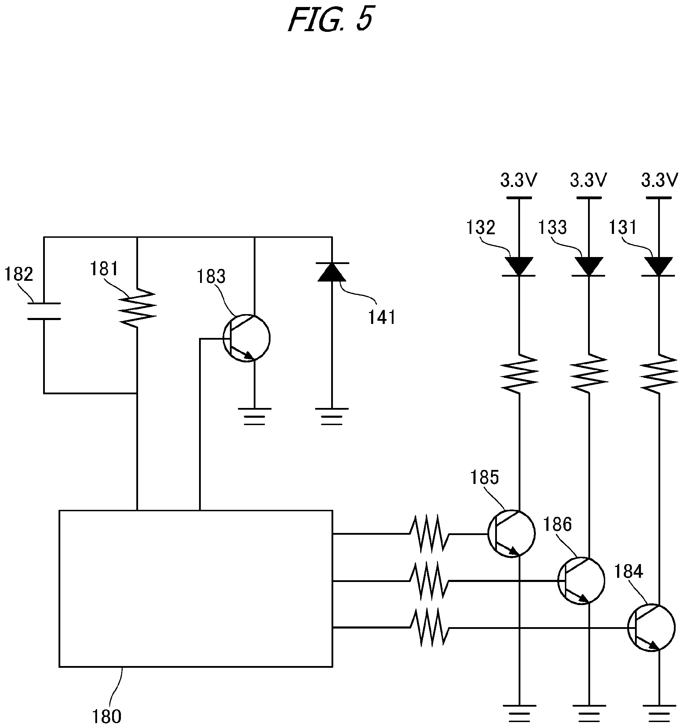

Fig. 5 is a circuit diagram of a part of the pulse wave signal measurement device 100 according to the present embodiment. The pulse wave signal measurement device 100 includes a microcomputer 180 constituting the controller 110 and the memory 120. The microcomputer 180 operates when power is supplied by a power supply not depicted (for example, a secondary battery) included in the pulse wave signal measurement device 100. One end side (output terminal) of the PD 141 is connected to the microcomputer 180 via an RC parallel circuit (R=680 kQ, C=3 nF) in which a resistor 181 and a capacitor 182 are connected in parallel, and the other end side of the PD 141 is connected to the ground (grounded). In addition, the pulse wave signal measurement device 100 includes a transistor 183 (NPN type). In the transistor 183, the collector terminal is connected to the output terminal of the PD 141, the emitter terminal is connected to the ground, and the base terminal is connected to the microcomputer 180. The transistor 183 is an example of a switching element that switches between a connected state in which the output terminal of the PD 141 is connected to the ground and a nonconnected state in which the output terminal of the PD 141 is disconnected from the ground.

In addition, the pulse wave signal measurement device 100 according to the present embodiment acquires received light data for one cycle by emitting light having different wavelengths in the order of the third LED 133, the second LED 132, and the first LED 131 within a predetermined time (100 milliseconds). The transistor 183 connects the output terminal of the PD 141 to the ground after the first LED 131 emits light in a certain cycle, and disconnects the output terminal of the PD 141 from the ground before the third LED 133 emits light in a cycle after a certain cycle. Thereby, the output signal of the PD 141 can be reset once every cycle. Here, the switching control of the transistor 183 is executed by the microcomputer 180. The control program for executing this control is stored in the storage unit (the memory 120 depicted in Fig. 1) of the microcomputer 180.

In addition, as depicted in Fig. 5, anode terminals of the first LED 131, the second LED 132, the third LED 133 are connected to a power supply circuit (not depicted) that applies a DC voltage of 3.3 V, and their cathode terminals are connected to collector terminals of transistors 184 to 186 (NPN type) via a resistor. All emitter terminals of the transistors 184 to 186 are connected to the ground, and all base terminals of the transistors 184 to 186 are connected to the microcomputer 180 via a resistor. The microcomputer 180 applies a voltage to each base terminal of the transistors 184 to 186 according to a light-emitting timing of the first LED 131, the second LED 132, and the third LED 133, and a DC voltage of 3.3 V is applied to each LED. Thereby, the pulse wave signal measurement device 100 can execute light emission by the first LED 131, the second LED 132, and the third LED 133 at predetermined timings. Here, the control program for executing this control is stored in the storage unit (the memory 120 depicted in Fig. 1) of the microcomputer 180.

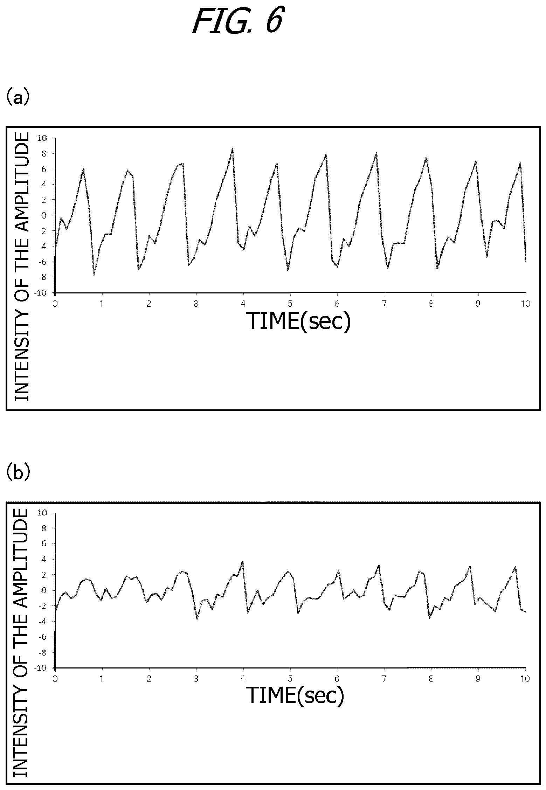

Next, a pulse wave signal measured by the pulse wave signal measurement device 100 will be described with reference to Fig. 6(a), Fig. 6(b). When the pulse wave signal measurement device 100 measures a pulse wave, the magnitude of the force that compresses the measurement site (hereinafter simply referred to as a "pressure value") affects the measurement result. In the present embodiment, since the pulse wave signal measurement device 100 emits light to the ventral side of the thumb and the light is received on the ventral side of the thumb, the pressure value on the ventral side of the thumb affects the measurement result.

Fig. 6(a) is a graph depicting an example of the pulse wave signal measured when the pressure value for the measurement site is appropriate. Fig. 6(b) is a graph depicting an example of the pulse wave signal measured when the pressure value for the measurement site is inappropriate. In the graphs of Fig. 6(a) and Fig. 6(b), the horizontal axis represents time (seconds), and the vertical axis represents the intensity (arbitrary unit) of the amplitude. As depicted in Fig. 6(a) and Fig. 6(b), it can be understood that, when the pressure value for the measurement site is appropriate, a pulse wave signal in a favorable beat state can be measured.

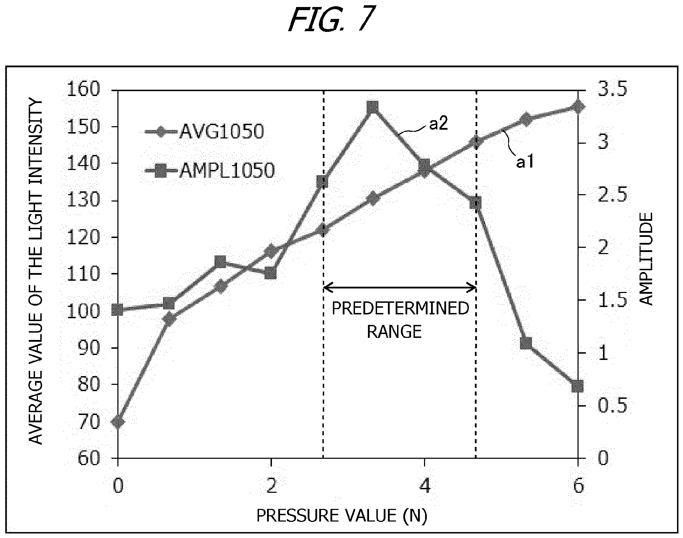

Next, an appropriate range of the pressure value for the measurement site will be described with reference to Fig. 7. Fig. 7 is a graph illustrating an appropriate range (hereinafter referred to as a "predetermined range") of the pressure value for the measurement site. The graph of Fig. 7 depicts the intensity of light and the magnitude of the amplitude of light received from the measurement site when the light having a peak wavelength of 1,050 nm is emitted while the measurement site is compressed. In the graph of Fig. 7, the horizontal axis represents the pressure value (N), the left vertical axis represents an average value (arbitrary unit) of the light intensity of the received light, and the right vertical axis represents the amplitude (arbitrary unit) of the received light. In addition, in the graph of Fig. 7, the line a1 depicts the change in the average value of the light intensity with respect to the pressure value, and the line a2 depicts the change in the amplitude with respect to the pressure value.

The blood vessels repeatedly contract and expand according to beating of the heart, and the blood volume in the blood vessels fluctuates according to the contraction and expansion. The pulse wave signal indicates the change in the intensity of light that is emitted to the measurement site and has passed through the blood vessels in the measurement site according to fluctuation in the blood volume. The intensity of light that has passed through the blood vessels decreases as the blood volume through which light passes increases, and increases as the blood volume through which light passes decreases. Therefore, a pressure is applied to the measurement site so that the difference between the maximum value and the minimum value of the blood volume in the blood vessels, which varies according to the beating, becomes relatively large, and thus a favorable pulse wave signal can be measured.

In the present embodiment, the predetermined range of the pressure value is set to a range in which the amplitude is 2 or more. As depicted in the graph of Fig. 7, the predetermined range of the pressure value is set to, for example, a range of about 2.7 N to about 4.7 N. Accordingly, the predetermined range of the pressure value can be monitored based on the amplitude of the received light (the amplitude of the pulse wave signal). In the blood component measurement system 1 according to the present embodiment, the pulse wave signal measurement device 100 sends the measured pulse wave signal to the terminal device 200 in real time. The terminal device 200 performs a process of analyzing the pulse wave signal received from the pulse wave signal measurement device 100 and determining a pressure condition of the measurement site.

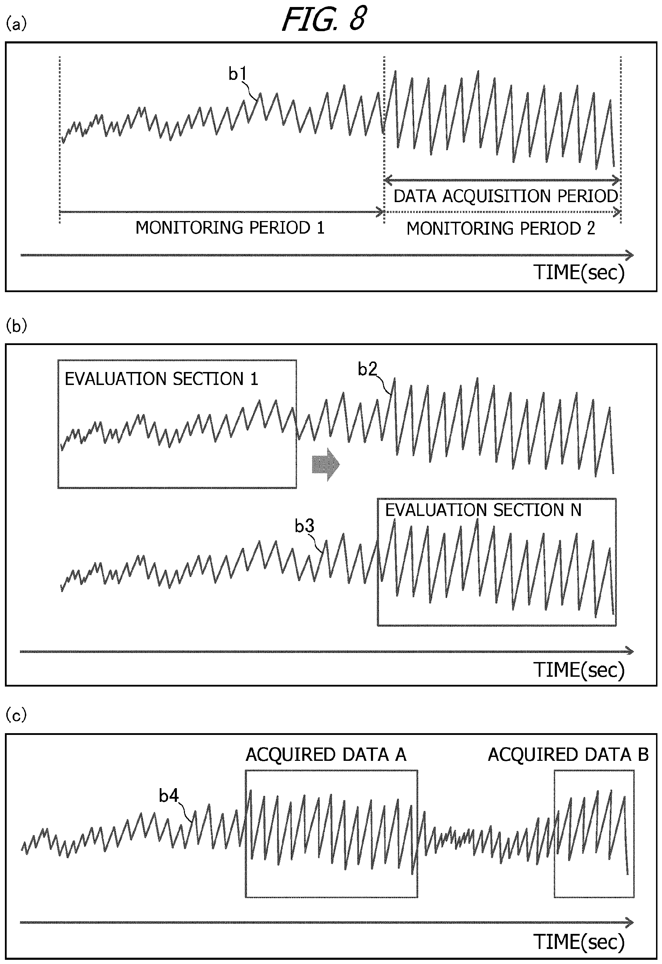

Next, a method of monitoring a pressure value will be described with reference to Fig. 8(a) to Fig. 8(c). Fig. 8(a) is a graph conceptually illustrating an example of the method of monitoring a pressure value according to the present embodiment. In the graph of Fig. 8(a), the horizontal axis represents time (seconds), and the line b1 depicts the pulse wave signal. In this example, a monitoring period 1 is provided as a pulse wave signal measurement period until the pressure value for the measurement site is within a predetermined range. In the monitoring period 1, the pulse wave signal measurement device 100 turns the first LED 131 on to emit light having a peak wavelength of 1,050 nm to the subject's thumb, the light that has passed through the blood in the thumb is received by the PD 141, and a pulse wave signal is measured. In addition, the pulse wave signal measurement device 100 sends the measured pulse wave signal to the terminal device 200 in real time, and the terminal device 200 analyzes the pulse wave signal and determines whether the pressure value for the measurement site is within a predetermined range. When it is determined that the pressure value for the measurement site is within the predetermined range, the terminal device 200 causes the pulse wave signal measurement device 100 to start measurement of a pulse wave signal for blood component measurement and starts a monitoring period 2 (an example of the "predetermined period"). The monitoring period 2 is a data acquisition period for blood component measurement (about 20 seconds), and the pulse wave signal measurement device 100 sequentially turns the first LED 131 to the third LED 133 on in the monitoring period 2 and measures a pulse wave signal. The terminal device 200 calculates the blood TG value and HbAlc value based on the pulse wave signal acquired in the monitoring period 2. According to the blood component measurement system 1 of the present embodiment, since the blood TG value and HbAlc value are calculated based on the pulse wave signal measured when the pressure value for the measurement site is within the predetermined range, it is possible to improve the measurement accuracy of the blood component concentration.

In this manner, in the monitoring period 1, the first LED 131 is turned on. This is because, in the monitoring period 1, the pulse wave signal for pressure value monitoring instead of the pulse wave signal for blood component measurement is measured, the number of received light data items can be increased by emitting light with one LED rather than sequentially turning a plurality of LEDs on and acquiring received light data, and thus it is possible to analyze the pressure value in more detail than in the monitoring period 2. In addition, the terminal device 200 monitors the pressure value by analyzing the pulse wave signal obtained from light emitted from the first LED 131 among pulse wave signals for blood component measurement also in the monitoring period 2.

Fig. 8(b) is a graph conceptually illustrating an example of the method of monitoring a pressure value according to the present embodiment. In the graph of Fig. 8(b), the horizontal axis represents time (seconds), and the line b2 and the line b3 depict the pulse wave signal. The line b2 and the line b3 are drawn in two upper and lower stages for convenience of explanation, and both depict the same pulse wave signal. In this example, while the evaluation section of the unit time is shifted for each time section, the pressure value is repeatedly evaluated, and the pulse wave signal for blood component measurement is acquired after the evaluation section in which the pressure value is within the predetermined range. Here, in this example, in the evaluation section before the pressure value is within the predetermined range, the first LED 131 is turned on to measure the pulse wave signal for pressure value monitoring, and in the evaluation section after the pressure value is within the predetermined range, the first LED 131 to the third LED 133 are sequentially turned on to measure the pulse wave signal for blood component measurement. In addition, as depicted in the graph of Fig. 8(b), the pressure value is outside of the predetermined range in an evaluation section 1, but when the terminal device 200 determines that the pressure value is within the predetermined range in the evaluation section N (N is a natural number of 2 or more), the pulse wave signal measurement device 100 measures the pulse wave signal for blood component measurement, for example, for about 20 seconds (an example of the "predetermined period") after the evaluation section N starts. The terminal device 200 calculates the blood TG value and HbAlc value based on the pulse wave signal for blood component measurement for about 20 seconds. According to the blood component measurement system 1 of the present embodiment, since the blood TG value and HbAlc value are calculated based on the pulse wave signal measured when the pressure value for the measurement site is within the predetermined range, it is possible to improve the measurement accuracy of the blood component concentration.

Fig. 8(c) is a graph conceptually illustrating an example of the method of monitoring a pressure value according to the present embodiment. In the graph of Fig. 8(c), the horizontal axis represents time (seconds), and the line b4 depicts the pulse wave signal. In this example, a case in which the pressure value changes before acquisition of the pulse wave signal for blood component measurement is completed will be described. Here, in this example, in the period before the pressure value first falls within the predetermined range, the first LED 131 is turned on to measure the pulse wave signal for pressure value monitoring, and in the period after the pressure value first falls within the predetermined range, the first LED 131 to the third LED 133 are sequentially turned on to measure the pulse wave signal for blood component measurement. For example, as depicted in the graph of Fig. 8(c), when the terminal device 200 determines that the pressure value first falls within the predetermined range based on the pulse wave signal for pressure value monitoring, the pulse wave signal measurement device 100 measures the pulse wave signal for blood component measurement (acquired data A in Fig. 8(c)). The terminal device 200 continues to monitor the pressure value based on the acquired data A, and when it determines that the pressure value is outside of the predetermined range before the measurement time of the acquired data A reaches the predetermined period (about 20 seconds), the pulse wave signal (in Fig. 8(c), acquired data B) acquired after it is determined that the pressure value is within the predetermined range is used as the pulse wave signal for blood component measurement. The terminal device 200 causes the pulse wave signal measurement device 100 to measure the pulse wave signal for blood component measurement so that the total measurement time of the acquired data A and the acquired data B becomes a predetermined period, and calculates the blood TG value and HbAlc value based on the pulse wave signal of the acquired data A and the acquired data B. As in this example, the pulse wave signal for blood component measurement may be acquired as two or more divided data. The blood component measurement system 1 according to the present embodiment constantly monitors the pressure value in order to calculate the blood component concentration based on the pulse wave signal while the pressure value within the predetermined range is applied to the measurement site. Thereby, in the blood component measurement system 1, even if the pressure value for the measurement site changes outside the predetermined range during measurement of the pulse wave signal for blood component measurement, the processing unit 211 determines the change in the pressure value, and the calculation unit 212 selectively uses the pulse wave signal acquired in the period in which the pressure value for the measurement site is within the predetermined range again and calculates the blood component concentration. Thereby, the blood component measurement system 1 can improve the measurement accuracy of the blood component concentration.

Next, a processing method of determining the pressure condition of the measurement site will be described in detail. In the present embodiment, the pressure condition of the measurement site is determined by performing an analysis in which the pressure value is quantified. In the present embodiment, for an analysis method of quantifying the pressure value, a total of four methods including frequency analysis of the pulse wave signal, counting of inflection points of the pulse wave signal, comparison with a reference pattern obtained from shapes of a plurality of beats in the pulse wave signal, and comparison with a sawtooth wave obtained from the shape of a single beat in the pulse wave signal will be exemplified.

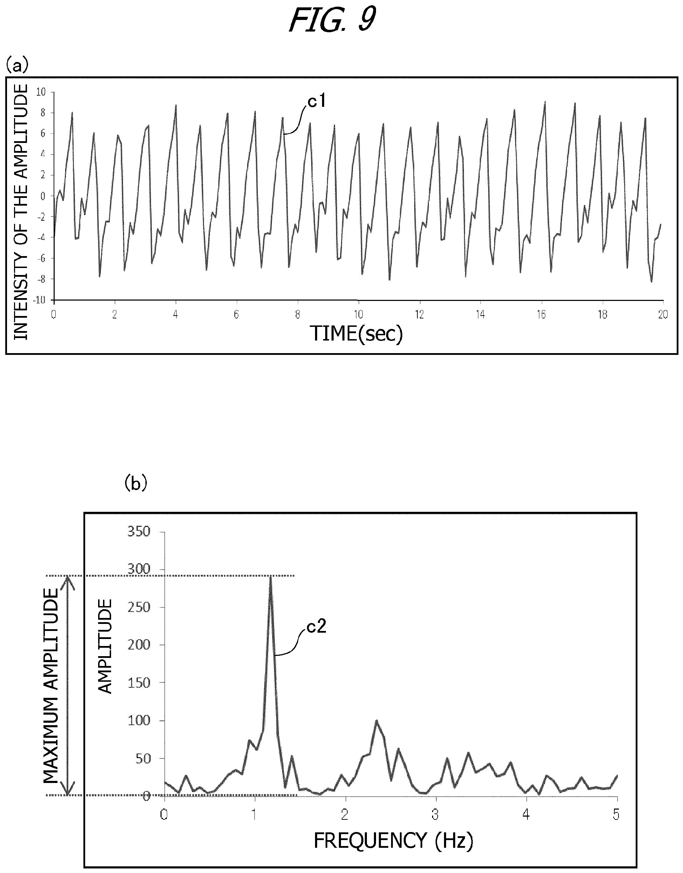

First, frequency analysis will be described. Fig. 9(a) is a graph depicting an example of the pulse wave signal measured by the pulse wave signal measurement device 100 when the pressure value for the measurement site is within the predetermined range. In the graph of Fig. 9(a), the horizontal axis represents time (seconds), the vertical axis represents the intensity (arbitrary unit) of the amplitude, and the line c1 depicts the pulse wave signal. Fig. 9(b) is a graph depicting the amplitude spectrum of a signal component for each frequency of a pulse wave signal obtained by Fourier transforming the pulse wave signal depicted in Fig. 9(a) for each unit time. In the graph of Fig. 9(b), the horizontal axis represents frequency (Hz), the vertical axis represents amplitude (arbitrary unit), and the line c2 depicts the amplitude spectrum.

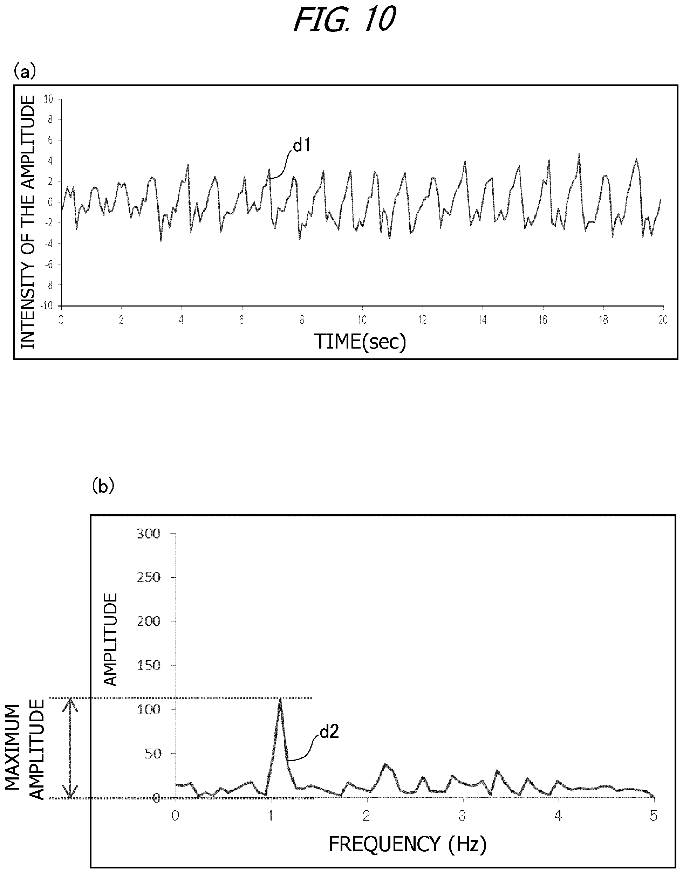

On the other hand, Fig. 10(a) is a graph depicting an example of the pulse wave signal measured by the pulse wave signal measurement device 100 when the pressure value for the measurement site is outside of the predetermined range. In the graph of Fig. 10(a), the horizontal axis represents time (seconds), the vertical axis represents the intensity (arbitrary unit) of the amplitude, and the line d1 depicts the pulse wave signal. Fig. 10(b) is a graph depicting the amplitude spectrum of a signal component for each frequency of a pulse wave signal obtained by Fourier transforming the pulse wave signal depicted in Fig. 10(a) for each unit time. In the graph of Fig. 10(b), the horizontal axis represents frequency (Hz), the vertical axis represents amplitude (arbitrary unit), and the line d2 depicts the amplitude spectrum.

As depicted in Fig. 9(b) and Fig. 10(b), the maximum amplitude after a Fourier transform (the amplitude corresponding to the frequency of pulsation) is larger in the pulse wave signal measured when the pressure value for the measurement site is within the predetermined range than the pulse wave signal measured when the pressure value for the measurement site is outside of the predetermined range. Accordingly, since there is a correlation between the pressure value for the measurement site and the maximum amplitude of the pulse wave signal after a Fourier transform, the correlation between the predetermined range of the pressure value and the maximum amplitude of the pulse wave signal after a Fourier transform can be measured in advance, and the pressure value can be quantified from the maximum amplitude of the pulse wave signal after a Fourier transform. Here, in the frequency analysis in the present embodiment, the pulse wave signal may be normalized by the amplitude of the beat before it is subjected to a Fourier transform. Even if such normalization is performed, the correlation between the maximum amplitude of the pulse wave signal after a Fourier transform and the pressure value for the measurement site does not change.

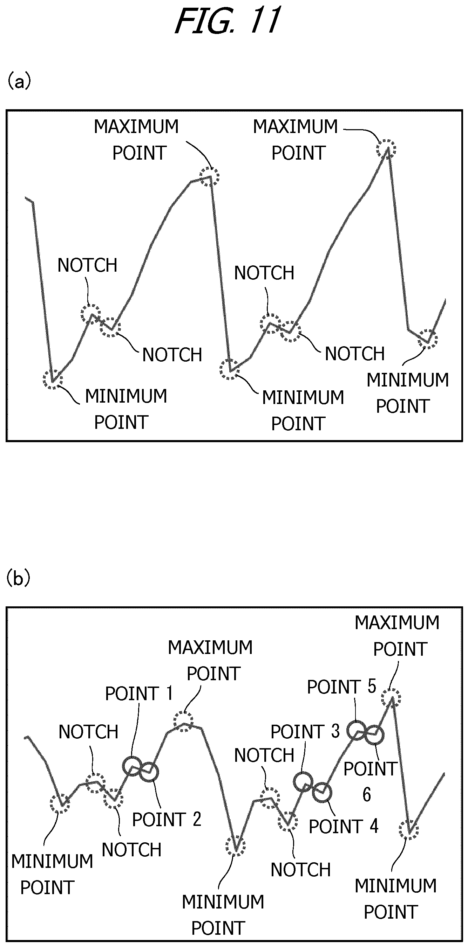

Next, counting of inflection points of the pulse wave signal will be described. Fig. 11(a) is a graph depicting an example of the pulse wave signal measured by the pulse wave signal measurement device 100 when the pressure value for the measurement site is within the predetermined range. Fig. 11(b) is a graph depicting an example of the pulse wave signal measured by the pulse wave signal measurement device 100 when the pressure value for the measurement site is outside of the predetermined range. Fig. 11(a) and Fig. 11(b) depict pulse wave signals for two beats.

In one beat for the pulse wave signal, there are a set of minimum point and maximum point, and a notch as inflection points. Here, the pulse wave signal is formed by overlapping a forward ejected wave created by ejection of the blood and a backward reflected wave created by reflection from the periphery, and the depression at the boundary between the ejected wave and the reflected wave is defined as a notch.

In counting of inflection points of the pulse wave signal in the present embodiment, the minimum point, the maximum point and the notch are excluded from the inflection points to be counted. As depicted in Fig. 11(a), in the pulse wave signal measured when the pressure value for the measurement site is within the predetermined range, there are no inflection points other than the minimum point, the maximum point and the notch, and the number of inflection points to be counted is "0". On the other hand, as depicted in Fig. 11(b), in the pulse wave signal measured when the pressure value for the measurement site is outside of the predetermined range, there are inflection points (Fig. 11(b), point 1 to point 6) other than the minimum point, the maximum point and the notch, and the number of inflection points to be counted is "6".

Here, in a favorable pulse wave signal, there are few inflection points to be counted and in an ideal pulse wave signal, the number of inflection points to be counted is "0". On the other hand, in a poor pulse wave signal, there are a relatively large number of inflection points to be counted. In this manner, a correlation is observed between the favorability of the pulse wave signal and the inflection points to be counted. Therefore, a correlation is observed between the pulse wave signal measured when the pressure value for the measurement site is within the predetermined range and each pulse wave signal measured when the pressure value is outside of the predetermined range with inflection points to be counted. In counting of inflection points of the pulse wave signal in the present embodiment, for example, a correlation between the number of inflection points to be counted in the pulse wave signal for five beats and the pressure value for the measurement site when the pulse wave signal is measured can be obtained in advance, and the pressure value can be quantified by the number of inflection points.

Next, comparison with a reference pattern obtained from shapes of a plurality of beats (hereinafter simply referred to as a "comparison method 1") will be described. In the comparison method 1, the pulse wave signal is divided in a plurality of beats, the number of beats in a unit time is superimposed, and the reference beat is calculated by averaging. Thereby, in the comparison method 1, the total value of Euclidean distances between the reference beat and the beat to be evaluated is calculated. Here, in the comparison method 1, the pulse wave signals of a plurality of beats can be analyzed by sequentially shifting the beats from when the reference beat is calculated and the beats to be evaluated one beat at a time.

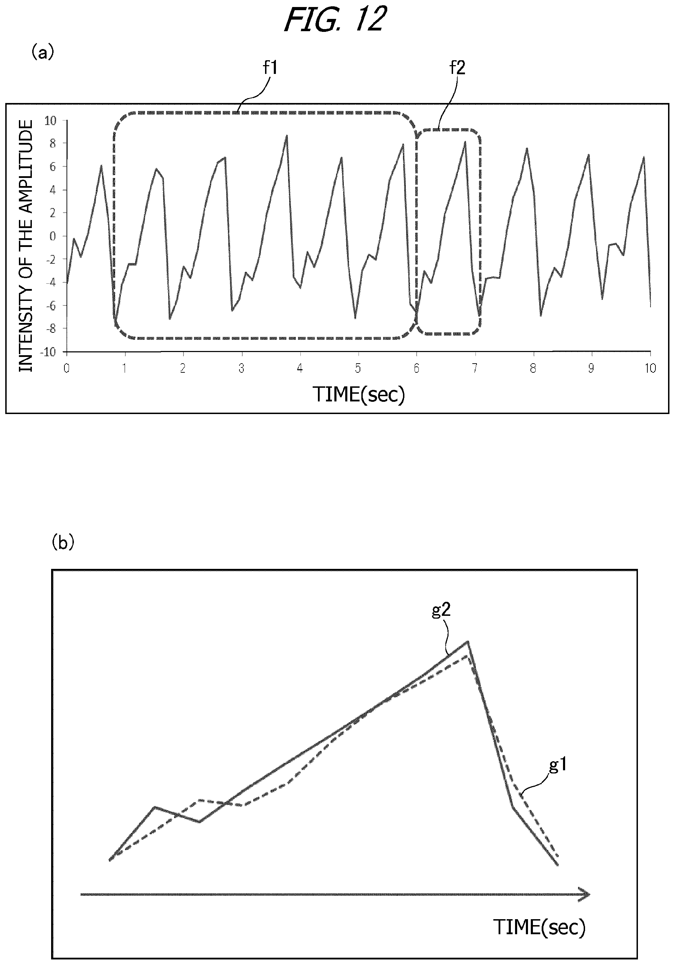

Fig. 12(a) is a graph depicting an example of the pulse wave signal. In the graph of Fig. 12(a), the horizontal axis represents time (seconds), and the vertical axis represents the intensity (arbitrary unit) of the amplitude. In this example, a method in which five beats surrounded by the dotted line f1 depicted in Fig. 12(a) are superimposed to calculate a reference beat and the beat surrounded by the dotted line f2 depicted in Fig. 12(a) is evaluated will be exemplified.

Fig. 12(b) is a graph in which the reference beat and the beat to be evaluated are superimposed. In the graph of Fig. 12(b), the horizontal axis represents time (seconds), the dotted line g1 depicts the reference beat, and the line g2 depicts the beat to be evaluated.

The total value of Euclidean distances between the reference beat and the beat to be evaluated becomes smaller when the pulse wave signal becomes better and becomes larger when the pulse wave signal becomes poorer. Accordingly, a correlation is observed between the favorability of the pulse wave signal and the total value of the Euclidean distance. Therefore, a correlation is observed between the pulse wave signal measured when the pressure value for the measurement site is within the predetermined range and each pulse wave signal measured when the pressure value is outside of the predetermined range with the total value of the Euclidean distance. In the comparison method 1 in the present embodiment, a correlation between the total value of the Euclidean distance and the pressure value for the measurement site when the pulse wave signal is measured can be obtained in advance, and the pressure value can be quantified by the total value of the Euclidean distance.

Next, comparison with a sawtooth wave obtained from the shape of a single beat (hereinafter simply referred to as a "comparison method 2") will be described. In the comparison method 2, the shape obtained by connecting the valleys and peaks of the pulse wave signal is regarded as an optimal pulse wave pattern, the magnitude of the Euclidean distance, which is the difference obtained by comparing this pattern with each individual beat, is compared, and thus the pulse wave signal is evaluated.

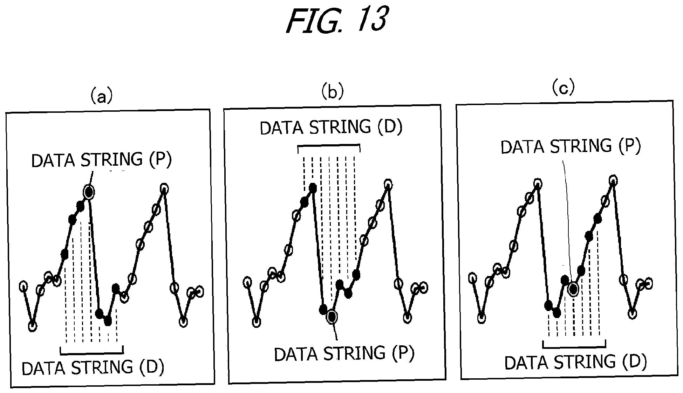

Fig. 13(a) to Fig. 13(c) depict pulse wave signals separated by a unit time. Here, in the pulse wave signal depicted in Fig. 13(a), data (P) at a certain time and a data string (D) composed of several points before and after the data (P) are compared. Here, a total of 7 points of data, with 3 points each before and after a certain time, is used as the data string (D). When the data (P) matches the maximum value of the data string (D), the data (P) can be regarded as a peak in each individual beat.

Next, the valley in the beat can be extracted by sequentially moving the time of interest. For example, when the data (P) depicted in Fig. 13(b) matches the minimum value of the data string (D), the data (P) can be regarded as a valley in each individual beat.

On the other hand, as depicted in Fig. 13(c), when the data (P) does not match the peak extracted in Fig. 13(a) or the valley extracted in Fig. 13(b), it can be considered that the data (P) is neither a peak nor a valley.

By sequentially moving the time of interest in this manner, it is possible to dynamically and almost immediately extract peaks and valleys of each individual beat in the pulse wave signal from the pulse wave signal. The rectangle obtained by connecting the peaks and valleys extracted in this manner can be used as a comparison pattern when the shape of the pulse wave signal is evaluated.

The pressure value for the measurement site can be quantified using the comparison pattern obtained by the above method. In the following examples, the pulse wave signal is evaluated by the total value of the Euclidean distances of the measured pulse wave signal and the comparison pattern.

### (Comparison method 2-1)

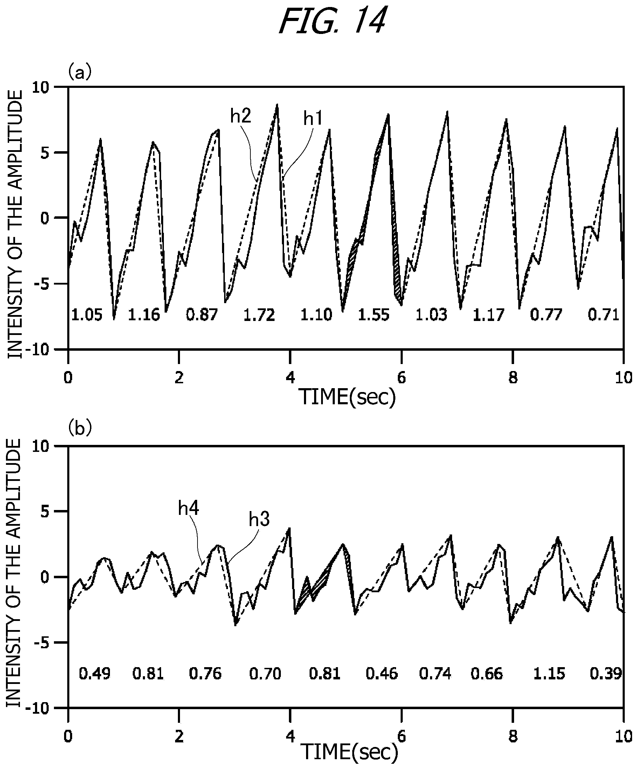

Fig. 14(a) is a graph depicting an example of a favorable pulse wave signal measured when the pressure value for the measurement site is appropriate. Fig. 14(b) is a graph depicting an example of a poor pulse wave signal measured when the pressure value for the measurement site is inappropriate. In the graphs of Fig. 14(a) and Fig. 14(b), the horizontal axis represents time (seconds), the vertical axis represents the intensity (arbitrary unit) of the amplitude, the solid lines h1 and h3 depict the measured pulse wave signals, and the dotted lines h2 and h4 depict comparison patterns. In a comparison method 2-1, based on the Euclidean distance between the favorable pulse wave signal and the poor pulse wave signal, and each comparison pattern, the area surrounded by the solid lines h1 and h3 and the dotted lines h2 and h4 in the graphs of Fig. 14(a) and Fig. 14(b) (in Fig. 14, indicated by upward sloping hatching (in this example, only one-beat is depicted)) is calculated. In the graph of Fig. 14(a) and the graph of Fig. 14(b), the area value is larger in the graph of Fig. 14(a). The average value of the area for 10 beats is 1.11 in the graph depicted in Fig. 14(a) and 0.70 in the graph depicted in Fig. 14(b). The total value of the Euclidean distances between the solid line h1 and the dotted line h2 is smaller than the total value of the Euclidean distances between the solid line h3 and the dotted line h4, but since the favorable pulse wave signal has a larger amplitude than the poor pulse wave signal, the area value is larger in the graph of Fig. 14(a). Here, the area values for each individual beat are depicted in the graphs of Fig. 14(a) and Fig. 14(b).

### (Comparison method 2-2)

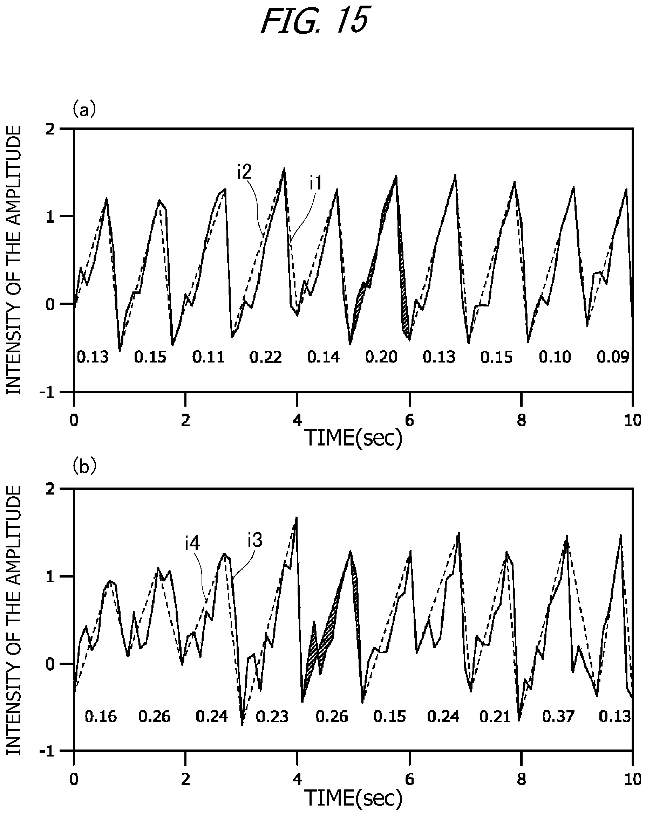

Fig. 15(a) is a graph depicting an example of the favorable pulse wave signal measured when the pressure value for the measurement site is appropriate. Fig. 15(b) is a graph depicting an example of the poor pulse wave signal measured when the pressure value for the measurement site is inappropriate. In the graphs of Fig. 15(a) and Fig. 15(b), the horizontal axis represents time (seconds), the vertical axis represents the intensity (arbitrary unit) of the amplitude, the solid lines i1 and i3 depict the measured pulse wave signals, and the dotted lines i2 and i4 depict comparison patterns. Here, the pulse wave signals depicted in Fig. 15(a) and Fig. 15(b) are normalized by the magnitude of the amplitude of the beat.

In a comparison method 2-2, as in the comparison method 2-1, based on the Euclidean distance between each pulse wave signal and each comparison pattern, the area surrounded by the solid lines i1 and i3 and the dotted lines i2 and i4 in the graphs of Fig. 15(a) and Fig. 15(b) (in Fig. 15, indicated by upward sloping hatching (in this example, only one-beat is depicted)) is calculated. In the graph of Fig. 15(a) and the graph of Fig. 15(b), the area value is larger in the graph of Fig. 15(b). The average value of the area for 10 beats is 0.14 in the graph depicted in Fig. 15(a) and 0.23 in the graph depicted in Fig. 15(b). In the comparison method 2-2, the size of the area can be used as an index of the pulse wave signal. Here, the area values for each individual beat are depicted in the graphs of Fig. 15(a) and Fig. 15(b).

Here, in the comparison method 2-2, the problem in the comparison method 2-1 is solved by normalizing the amplitude from the pulse wave signal for 10 beats, but in order to acquire the pulse wave signal for 10 beats, if the heart rate is 60 beats/min, the measurement time of the pulse wave signal needs to be about 10 seconds, and the normalization method is not desirable when the pressure condition of the measurement site is monitored (for example, the monitoring period 1 depicted in Fig. 8(a)). Therefore, it is better to normalize the pulse wave signal from as few beats as possible. On the other hand, since the accuracy of normalization is inferior when normalization is performed from one beat, it is better to determine the number of beats used for normalization in consideration of both accuracy and immediacy.

### (Comparison method 2-3)

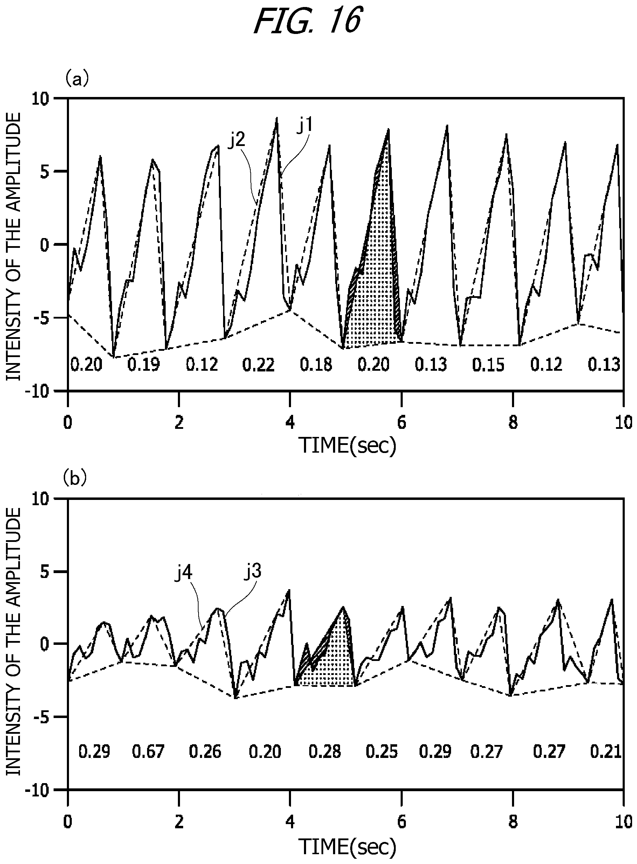

Fig. 16(a) is a graph depicting an example of the favorable pulse wave signal measured when the pressure value for the measurement site is appropriate. Fig. 16(b) is a graph depicting an example of the poor pulse wave signal measured when the pressure value for the measurement site is inappropriate. In the graphs of Fig. 16(a) and Fig. 16(b), the horizontal axis represents time (seconds), the vertical axis represents the intensity (arbitrary unit) of the amplitude, the solid lines j1 and j3 depict the measured pulse wave signals, and the dotted lines j2 and j4 depict comparison patterns. Here, the pulse wave signals depicted in Fig. 16(a) and Fig. 16(b) are normalized by the magnitude of the amplitude of the beat.

In a comparison method 2-3, unlike the comparison method 2-1 and the comparison method 2-2, the area of the triangle formed by three points of a beat valley, a peak, and a valley (in Fig. 16(a) and Fig. 16(b), the area indicated by dot hatching) is calculated, and the proportion of the area (in Fig. 16, the area indicated by upward sloping hatching) is calculated, and thus the deviation from the comparison pattern can be quantified. In Fig. 16(a) and Fig. 16(b), how much each individual beat obtained by the comparison method 2-3 deviates from the above triangle is quantified and depicted. As the value is larger, the deviation of the measured pulse wave signal from the comparison pattern is larger. In addition, the average value of the deviations of the measured pulse wave signal from the comparison pattern calculated from 10 beats is 0.16 in the case depicted in Fig. 16(a), and 0.30 in the case depicted in Fig. 16(b).

The advantage of the comparison method 2-3 is that the state of the pulse wave signal can be immediately quantified since the comparison pattern is a one-beat triangle formed from the valleys and peaks of the beat. In addition, since the proportion of deviation of the beat from the triangle can be quantified, the state of the pulse wave signal can be scored in a range of 0 to 1 or as a percentage by subtracting the obtained value from 1. For example, in the case of the graph of Fig. 16(a), the score of the pulse wave signal obtained from 10 beats is 0.84 (84%), and in the case of the graph of Fig. 16(b), the score of the pulse wave signal obtained from 10 beats is 0.70 (70%).

In this manner, a correlation is observed between the favorability of the pulse wave signal and the total value of the Euclidean distance with the comparison pattern. Therefore, a correlation is observed between the pulse wave signal measured when the pressure value for the measurement site is within the predetermined range and each pulse wave signal measured when the pressure value is outside of the predetermined range with the total value of the Euclidean distance. In the comparison method 2 in the present embodiment, a correlation between the total value of the Euclidean distance and the pressure value for the measurement site when the pulse wave signal is measured can be obtained in advance, and the pressure value can be quantified by the total value of the Euclidean distance.

In addition, in the present embodiment, a method in which, when valleys and peaks are extracted from the pulse wave signal, the data (P) at the time of interest and the maximum values and the minimum values of the data string (D) are compared is exemplified using a total of 7 points, with 3 points before and 3 points after the time of interest, but the minimum point and the maximum point can be extracted by another method. For example, valleys and peaks may be extracted by comparing the magnitude relationships or differential values with data points before and after a certain time.

In addition, in the present embodiment, the Euclidean distance is calculated by taking the square root of the sum of squares of two values of deviation of the pulse wave signal from the comparison pattern, but the sum of absolute values of the values may be used to calculate the Euclidean distance.

Next, a measurement process executed by the blood component measurement system 1 according to the present embodiment will be described with reference to Fig. 17. Fig. 17 is a flowchart depicting a measurement process executed by the blood component measurement system 1. The subject inserts his or her thumb into the opening 172 of the pulse wave signal measurement device 100, turns the knob 173, and adjusts a pressing force of the thumb against the contact surface 170a. Then, the subject operates the terminal device 200 and starts blood component measurement.

First, in an OP 201, the terminal device 200 sends an instruction to measure a pulse wave signal used for pressure value determination to the pulse wave signal measurement device 100. When the pulse wave signal measurement device 100 receives the measurement instruction in the OP 201, it starts a process of an OP 101. In the OP 101, the pulse wave signal measurement device 100 turns the first LED 131 on, emits light having a peak wavelength of 1,050 nm to the subject's thumb, receives the light that has passed through the blood in the finger by the PD 141, and measures the pulse wave signal. The pulse wave signal measurement device 100 sends the measured pulse wave signal to the terminal device 200 in real time.

Then, in an OP 202 (an example of the "process of determining a pressure condition"), the processing unit 211 of the terminal device 200 analyzes the pulse wave signal and thus determines whether the pressure value for the measurement site is within the predetermined range. Examples of pulse wave signal analysis methods include the analysis method of quantifying the pressure value described above.

When the processing unit 211 determines in the OP 202 that the pressure value is not within the predetermined range, the process proceeds to an OP 203. In the OP 203, the terminal device 200 instructs the subject to correct the pressure value. For example, the terminal device 200 displays characters, images or the like that instruct the subject to correct the pressure value on the display 230. The subject turns the knob 173 and adjusts the pressing force of the thumb against the contact surface 170a while referring to characters or images displayed on the display 230. The blood component measurement system 1 repeatedly executes the processes of the OP 101, the OP 202, and the OP 203 until the pressure value reaches a predetermined range. The period during which the processes of the OP 101, the OP 202, and the OP 203 are repeatedly executed is the monitoring period 1 depicted in Fig. 8 (a) .

On the other hand, when the processing unit 211 determines in the OP 201 that the pressure value is within the predetermined range, the process proceeds to an OP 204. In the next OP 204, the terminal device 200 sends an instruction to measure the pulse wave signal for blood component measurement to the pulse wave signal measurement device 100.

When the pulse wave signal measurement device 100 receives the measurement instruction in the OP 204, the process proceeds to an OP 102. In the OP 102, the pulse wave signal measurement device 100 measures the pulse wave signal for blood component measurement. The pulse wave signal measurement device 100 emits light from the third LED 133, the second LED 132, and the first LED 131 in that order to the subject's thumb, receives the light that has passed through the blood in the finger by the PD 141, and acquires received light data. For example, the pulse wave signal measurement device 100 acquires received light data for 20 seconds (200 cycles) and uses the received light data for 20 seconds as a pulse wave signal. In addition, the pulse wave signal measurement device 100 sends the measured pulse wave signal to the terminal device 200 in real time. The period during which the pulse wave signal for 20 seconds is measured is the monitoring period 2 depicted in Fig. 8(a). The terminal device 200 receives the pulse wave signal in an OP 205, and performs a process of determining a pressure condition of the measurement site by analyzing the pulse wave signal obtained from light emitted from the first LED 131 among pulse wave signals for blood component measurement with the processing unit 211.

As described above, in the processes of the OPs 201 to 205, the terminal device 200 determines whether the pressure value for the measurement site is within the predetermined range when the processing unit 211 performs a process of determining a pressure condition of the measurement site. Then, the terminal device 200 continues to perform the process of determining a pressure condition on the pulse wave signal acquired after the processing unit 211 determines that the pressure value for the measurement site is within the predetermined range, and the calculation unit 212 calculates the blood component concentration in processes of an OP 206 to an OP 208.

In the next OP 206, the calculation unit 212 of the terminal device 200 calculates the absorbance corresponding to each wavelength from the pulse wave signal. For example, the variation width of the pulse wave signal corresponding to each wavelength can be appropriately converted to the absorbance. The calculation unit 212 calculates the absorbance of blood corresponding to emission of light having a wavelength of 1,050 nm (hereinafter referred to as a "first absorbance"), the absorbance of blood corresponding to emission of light having a wavelength of 1,300 nm (hereinafter referred to as a "second absorbance"), and the absorbance of blood corresponding to emission of light having a wavelength of 1,450 nm (hereinafter referred to as a "third absorbance") from the variation width of the pulse wave signal corresponding to emission of light having each wavelength.

In the next OP 207, the calculation unit 212 calculates the blood TG value from the first absorbance and the second absorbance. For example, the terminal device 200 calculates a non-invasive blood absorbance according to the difference between the first absorbance and the second absorbance and converts the non-invasive blood absorbance to the blood TG value using a predetermined conversion table. Thereby, the blood TG value is calculated.

In the next OP 208, the calculation unit 212 normalizes the third absorbance and converts the normalized third absorbance to an HbAlc value. As an example of normalization, for example, the third absorbance is divided by the first absorbance or the second absorbance. According to normalization of the third absorbance, the HbAlc value can be more accurately calculated without depending on the total hemoglobin concentration. In addition, as a method of converting the normalized third absorbance to the HbAlc value, for example, a method in which the memory 220 of the terminal device 200 stores a calibration curve data showing the relationship between the absorbance and the HbAlc value created in advance, and the HbAlc value is calculated by comparing the normalized third absorbance with the calibration curve is exemplified. Here, in order to create a calibration curve, it is preferable to use human hemoglobin and glycated hemoglobin to be measured.

In the next OP 209, the calculation unit 212 calculates the degree of reliability of the measured blood TG value and HbAlc value. The calculation unit 212 calculates the degree of reliability by scoring the pulse wave signal used for blood component calculation by, for example, the comparison method 2-2 or the comparison method 2-3 described above.

In the next OP 210, the calculation unit 212 determines whether the degree of reliability is equal to or larger than a predetermined value (for example, 0.8 (80%) or more). When the calculation unit 212 determines that the degree of reliability is less than a predetermined value, the process proceeds to an OP 212. In the OP 212, the terminal device 200 notifies the subject of re-measurement of the blood component using the display 230, and the process of the OP 201 is then executed again. Thereby, the blood component measurement system 1 re-measures the blood component concentration.

On the other hand, when the calculation unit 212 determines in the OP 210 that the degree of reliability is equal to or larger than a predetermined value, the process proceeds to an OP 211. In the OP 211, the terminal device 200 displays the blood TG value and HbAlc value and the degree of reliability as the measurement result on the display 230. In this manner, the terminal device 200 includes the display 230 that notifies of the blood component concentration when the degree of reliability is equal to or larger than a predetermined value and notifies of re-measurement of the blood component concentration when the degree of reliability is less than a predetermined value. Thereby, the subject can be notified of the blood TG value and HbAlc value.

According to the present embodiment, since an accurate pulse wave signal can be detected, it is possible to improve the measurement accuracy of the blood component concentration.

While the present embodiment has been described above, the configuration of the pulse wave signal measurement device 100, the process of calculating the blood TG value and HbAlc value and the like are not limited to the above embodiment, and various modifications can be made within the scope in which the identity with the technical idea of the present invention is not lost.

For example, in the above embodiment, in the period before the pressure value first falls within the predetermined range, the first LED 131 is turned on to measure the pulse wave signal for pressure value monitoring. However, the LED that is turned on in the period before the pressure value first falls within the predetermined range is not limited to the first LED 131, and may be the second LED 132 or the third LED 133. In addition, two or more LEDs may be turned on even in the period before the pressure value first falls within the predetermined range.

In addition, in the monitoring period 2 depicted in Fig. 8(a), the processing unit 211 of the terminal device 200 may perform a process of analyzing the pulse wave signal obtained from light emitted from the second LED 132 or the third LED 133 and determining a pressure condition of the measurement site.

In addition, while light with wavelengths in increasing order of biopermeability is emitted to the measurement site of the living body in the above embodiment, the biopermeability varies depending on the living body and the measurement site. Therefore, the wavelengths in increasing order of biopermeability are not limited to a wavelength of 1,450 nm, a wavelength of 1,300 nm, and a wavelength of 1,050 nm. Here, the control of emitting light with wavelengths in increasing order of biopermeability may be executed based on the control signal from the terminal device 200. For example, the terminal device 200 stores biopermeability information regarding wavelengths in various measurement sites of the living body, and the terminal device 200 may control the pulse wave signal measurement device 100 so that light in ascending order of biopermeability is emitted at the measurement site of the living body to be measured.

In addition, in the present embodiment, the terminal device 200 calculates the blood component concentration based on the pulse wave signal measured by the pulse wave signal measurement device 100, but the present invention is not limited thereto. The device for measuring the pulse wave signal and the device for calculating the blood component concentration may be the same or integrated. For example, when the device for measuring the pulse wave signal and the device for calculating the blood component concentration are the same, the pulse wave signal measurement device 100 performs a process of analyzing the pulse wave signal and determining a pressure condition of the measurement site and calculates the blood component concentration. In this case, the pulse wave signal measurement device 100 is an example of a computer in the present invention.

In addition, the terminal device 200 sends the pulse wave signal obtained by the pulse wave signal measurement device 100 to an external server via a communication line, the external server calculates the blood TG value and HbAlc value based on the pulse wave signal and sends information including the blood TG value and HbAlc value to the terminal device 200 via a communication line, and the terminal device 200 may display the blood TG value and HbAlc value.

In addition, while the reflected light method is used in the pulse wave signal measurement device 100 in the above embodiment, a transmitted light type may be used. The transmitted light type pulse wave signal measurement device 100 may have a configuration in which, for example, the irradiator 130 is disposed on the upper part cover 171 so that the subject's thumb 300 inserted from the opening 172 is interposed between the irradiator 130 and the light receiver 140, the irradiator 130 emits light from the back side (nail side) of the thumb 300, and the light that has passed through the thumb is received by the light receiver 140.

In addition, the number of LEDs included in the irradiator 130, the peak wavelengths of light emitted from the LEDs, and the arrangement pattern are not limited to those in the above embodiment. For example, when only the blood TG value is measured, the irradiator 130 may include the first LED 131 and the second LED 132. In addition, when only the HbAlc value is measured, the irradiator 130 may include either the first LED 131 or the second LED 132, and the third LED 133.

In addition, while the living body to be measured is a human in the above embodiment, the living body to be measured is not limited to the human. Specific examples of living bodies to be measured include mammals and birds. Among these, it is more preferable to include humans who may be diagnosed with diseases caused by hyperglycemia (for example, diabetes) or mammals or birds which can be pets or livestock as measurement targets.

In addition, while the blood TG value and HbAlc value are measured as the blood component concentration in the above embodiment, the concentrations of other blood components may be measured. For example, hemoglobin, glucose, cholesterols (total cholesterol, HDL- or LDL-cholesterol, free cholesterol), urea, bilirubin, lipoprotein, phospholipid, ethyl alcohol or the like in blood may be measured. In this case, light having a wavelength of which the absorbance changes depending on the concentration of each blood component is emitted to the living body, and the concentration of each blood component is calculated from the absorbance.

In addition, the method of quantifying the pressure value depicted in the above embodiment is an example, and the pressure value may be quantified by another analysis method.

In addition, in the above embodiment, the pressing force against the subject's finger is adjusted by turning the knob 173. However, the method of adjusting the pressing force is not limited thereto. For example, a band made of a hook-and-loop fastener may be provided, and the pressing force may be adjusted by re-tightening the band. In addition, the pressing force may be adjusted by simply applying the force to the subject. Alternatively, a pressing mechanism driven by a motor may be provided, and may automatically adjust the pressing force in response to a signal from the terminal device 200.

In addition, in the above embodiment, the terminal device 200 includes the display 230 as a notification unit, but the notification unit is not limited thereto. The terminal device 200 includes a speaker as a notification unit, and may notify of the blood component concentration, the degree of reliability, and re-measurement by voice. In addition, the terminal device 200 may include both the display 230 and the speaker as the notification unit.

Here, in order to stabilize the pressure value for the measurement site, an elastic member is disposed between the upper part cover 171 and the housing 170 so that the measurement site is interposed between the upper part cover 171 and the contact surface 170a, and the measurement site may exert a biasing force on the contact surface 170a.

In addition, the allowable range of the degree of reliability of the blood component concentration (being equal to or larger than a predetermined value) may be changed according to the condition of the living body to be measured. For example, when the living body to be measured has not eaten, the allowable range of the degree of reliability may be set narrower, or when the living body to be measured has eaten, the allowable range of the degree of reliability may be set wider. In addition, the allowable range of the degree of reliability of the blood component concentration may change according to the time that has elapsed since the living body to be measured ate. For example, as the time that has elapsed since the living body to be measured ate is longer, the allowable range of the degree of reliability may be set narrower.

In addition, an example in which three LEDs having different light emission wavelengths are used has been depicted in the above embodiment, but the number of required LEDs and the type of the light emission wavelength of the LED are appropriately changed by changing or increasing the blood component to be measured. In addition, in the pulse wave signal measurement device 100, a white LED or a halogen lamp may be used for the irradiator 130, and a spectroscope may be used for the light receiver 140. In such a case, according to the technique of the present disclosure, it is possible to improve the measurement accuracy of the blood component concentration.

### [Reference Signs List]

- 1: Blood component measurement system
- 100: Pulse wave signal measurement device
- 200: Terminal device

## Claims

1. A blood component measurement system that emits light to a measurement site of a living body while the measurement site is compressed, and calculates a concentration of a predetermined blood component based on a pulse wave signal acquired based on light received from the measurement site, the system comprising:
a processing unit that performs a process of analyzing the pulse wave signal and determining a pressure condition of the measurement site; and
a calculation unit that calculates a concentration of the blood component using the pulse wave signal in a period in which the pressure condition is determined.

2. The blood component measurement system according to claim 1,
wherein the calculation unit calculates a concentration of the blood component selectively using the pulse wave signal acquired in a period in which a pressure value for the measurement site is within a predetermined range.

3. The blood component measurement system according to claim 1,
wherein the calculation unit calculates a concentration of the blood component using the pulse wave signal acquired in a predetermined period and calculates a degree of reliability of the concentration of the blood component based on the result of the process during the predetermined period,
the blood component measurement system further comprising
a notification unit that notifies of the concentration of the blood component when the degree of reliability is equal to or larger than a predetermined value and notifies of re-measurement of the concentration of the blood component when the degree of reliability is less than the predetermined value.

4. The blood component measurement system according to any one of claims 1 to 3,
wherein the processing unit determines whether a pressure value for the measurement site is within a predetermined range by performing the process, and
continuously performs the process on the pulse wave signal acquired after it is determined that the pressure value for the measurement site is within the predetermined range, and
wherein the calculation unit calculates a concentration of the blood component using the pulse wave signal acquired after the processing unit determines that the pressure value for the measurement site is within the predetermined range.

5. A blood component measurement method in which light is emitted to a measurement site of a living body while the measurement site is compressed, and a concentration of a predetermined blood component is calculated based on a pulse wave signal acquired based on light received from te measurement site, the method comprising:
performing a process of analyzing the pulse wave signal and determining a pressure condition of the measurement site; and
calculating a concentration of the blood component using the pulse wave signal in a period in which the pressure condition is determined.

6. The blood component measurement method according to claim 5,
wherein the concentration of the blood component is calculated selectively using the pulse wave signal acquired in a period in which a pressure value for the measurement site is within a predetermined range.

7. The blood component measurement method according to claim 5,
wherein the concentration of the blood component is calculated using the pulse wave signal acquired in a predetermined period and a degree of reliability of the concentration of the blood component is calculated based on the result of the process during the predetermined period, and
wherein, when the degree of reliability is equal to or larger than a predetermined value, the concentration of the blood component is notified of, and when the degree of reliability is less than the predetermined value, re-measurement of the concentration of the blood component is notified of.

8. The blood component measurement method according to any one of claims 5 to 7,
wherein it is determined whether a pressure value for the measurement site is within a predetermined range by performing the process,
wherein the process is continuously performed on the pulse wave signal acquired after it is determined that the pressure value for the measurement site is within the predetermined range, and
wherein the concentration of the blood component is calculated using the pulse wave signal acquired after it is determined that the pressure value for the measurement site is within the predetermined range.

9. A blood component measurement program that is executed by a blood component measurement system that emits light to a measurement site of a living body while the measurement site is compressed, and calculates a concentration of a predetermined blood component based on a pulse wave signal acquired based on light received from the measurement site, the program causing a computer to execute:
performing a process of analyzing the pulse wave signal and determining a pressure condition of the measurement site; and
calculating a concentration of the blood component using the pulse wave signal in a period in which the pressure condition is determined.

10. The blood component measurement program according to claim 9, causing the computer to calculate a concentration of the blood component selectively using the pulse wave signal acquired in a period in which a pressure value for the measurement site is within a predetermined range.

11. The blood component measurement program according to claim 9, causing the computer to
calculate a concentration of the blood component using the pulse wave signal acquired in a predetermined period and calculate a degree of reliability of the concentration of the blood component based on the result of the process during the predetermined period, and
notify of the concentration of the blood component when the degree of reliability is equal to or larger than a predetermined value and notify of re-measurement of the concentration of the blood component when the degree of reliability is less than the predetermined value.

12. The blood component measurement program according to any one of claims 9 to 11, causing the computer to
determine whether a pressure value for the measurement site is within a predetermined range by performing the process,
continuously perform the process on the pulse wave signal acquired after it is determined that the pressure value for the measurement site is within the predetermined range, and
calculate the concentration of the blood component using the pulse wave signal acquired after it is determined that the pressure value for the measurement site is within the predetermined range.
